(19) Europäisches Patentamt
European Patent Office
Office européen des brevets

(11) EP 0 578 541 B1

(12) # FASCICULE DE BREVET EUROPEEN

(45) Date de publication et mention de la délivrance du brevet:
**03.12.1997 Bulletin 1997/49**

(51) Int. Cl.[6]: **C07C 2/30**

(21) Numéro de dépôt: **93401702.1**

(22) Date de dépôt: **30.06.1993**

(54) **Procédé de fabrication d'oléfines alpha légères par oligomérisation de l'éthylène**

Verfahren zur Oligomerisierung von leichten Alpha-Olefinen durch Oligomerisierung von Ethylen

Process for the preparation of light alpha-olefines by oligomerisation of ethylene

(84) Etats contractants désignés:
**BE DE FR GB IT NL**

(30) Priorité: **09.07.1992 FR 9208658**

(43) Date de publication de la demande:
**12.01.1994 Bulletin 1994/02**

(73) Titulaire:
**INSTITUT FRANCAIS DU PETROLE
92502 Rueil-Malmaison (FR)**

(72) Inventeurs:
- **Chauvin, Yves
F-78230 Le Pecq (FR)**
- **Commereuc, Dominique
F-92190 Meudon (FR)**
- **Hugues, Francois
F-69390 Vernaison (FR)**
- **Olivier, Hélène
F-92500 Rueil Malmaison (FR)**
- **Saussine, Lucien
F-78290 Croissy Sur Seine (FR)**

(74) Mandataire: **Andreeff, François
INSTITUT FRANCAIS DU PETROLE
4, avenue de Bois-Préau
92502 Rueil-Malmaison (FR)**

(56) Documents cités:
**EP-A- 0 241 596**
**DE-A- 2 044 343**
**US-A- 4 855 525**
**WO-A-91/02707**
**FR-A- 2 669 921**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.15.2/3.4

## Description

La présente invention concerne un procédé d'oligomérisation de l'éthylène en oléfines alpha légères, principalement en butène-1, hexène-1, octène-1 et décène-1.

Dans le brevet US 2 943 125, K. Ziegler a décrit une méthode pour dimériser l'éthylène en butène-1 au moyen d'un catalyseur obtenu par le mélange d'un trialkylaluminium et d'un tétraalcoolate de titane ou de zirconium.

L'oligomérisation de l'éthylène en oléfines alpha de poids moléculaires variés est bien connue et met en jeu soit une réaction de croissance de chaine stoechiométrique, par exemple à partir d'un composé organoaluminique, soit une réaction catalytique faisant appel à des métaux tels que le titane, le zirconium, le chrome, le nickel ou les terres rares, par exemple dans des recettes de type Ziegler.

De nombreux composés de zirconium ont été mis en oeuvre pour réaliser l'oligomérisation de l'éthylène en oléfines alpha, souvent associés à des coordinats variés.

On peut citer par exemple l'utilisation d'halogénures de zirconium associés à des esters, cétones, éthers, amines, nitriles, anhydrides, chlorures d'acides, amides ou aldéhydes, décrite dans les brevets US 4 855 525 et WO 91 02707, ou l'utilisation des mêmes halogénures de zirconium associés à des ligands sélectionnés parmis les groupes des composés soufrés, phosphorés ou azotés, décrite dans les brevets EP-A-241 596 et EP-A-328 728.

Les produits obtenus avec les formules catalytiques ci-dessus sont constitués principalement d'oléfines alpha ayant une longueur de chaîne située entre $C_{10}$ et $C_{18}$. Ces mélanges conviennent bien pour les utilisations jusque là dévolues à ces oligomères, plastifiants et détergents notamment.

Il est bien connu de l'homme de l'art que la plupart de ces catalyseurs conduisent à la formation, à côté des alpha oléfines souhaitées, de quantités plus ou moins importantes de polymère de haute masse moléculaire qui gène considérablement la mise en oeuvre.

Un brevet FR-A-2.669.921 décrit un procédé de conversion de l'éthylène en oléfines alpha légères avec un catalyseur à base de zirconate d'alkyle, éther et composé halogéné d'aluminium. Le déposent a cherché à améliorer la sélectivité du procédé et a modifié le composition catalytique.

Il a été trouvé selon la présente invention que les catalyseurs obtenus en mélangeant un composé de zirconium avec au moins un composé organique choisi parmi la classe des acétals d'aldèhydes et des cétals de cétones, et avec au moins un composé particulier d'aluminium, présentent une sélectivité inattendue pour la formation des oligomères inférieurs, principalement le butène-1, l'hexène-1, l'octène-1 et le décène-1, qui trouvent une utilisation comme comonomères avec l'éthylène dans la fabrication du polyéthylène basse densité linéaire, ou comme base de départ pour des huiles lubrifiantes de synthèse.

Outre l'amélioration de la sélectivité pour les alpha oléfines légères, les catalyseurs décrits dans la présente invention ont également pour but de réduire à une quantité très faible le polymère sous-produit.

Les composés de zirconium utilisés dans l'invention répondent à la formule générale :

$ZrX_xY_yO_z$, dans laquelle X est un atome de chlore ou de brome et Y est un radical choisi parmi les groupes alkoxy $RO^-$, amido $R_2N$-, ou carboxylate $RCOO^-$, R étant un radical hydrocarbyl, de préférence alkyl, comprenant de 1 à 30 atomes de carbone. x et y peuvent prendre les valeurs entières de 0 à 4, et z est égal à 0 ou 0,5, la somme : $x + y + 2z$ étant égale à 4. On peut citer à titre d'exemples les halogénures de zirconium tels que le tétrachlorure de zirconium $ZrCl_4$, le tétrabromure de zirconium $ZrBr_4$, les alcoolates tels que le tétrapropylate de zirconium $Zr(OC_3H_7)_4$, le tétrabutylate de zirconium $Zr(OC_4H_9)_4$, les carboxylates tels que le tétra-éthyl-2-hexanoate de zirconium $Zr(OCOC_7H_{15})_4$ ou les oxo-carboxylates comme l' oxo-hexaéthyl-2-hexanoate de dizir-conium $[Zr(OCOC_7H_{15})_3]_2$ O.

Les composés organiques choisis parmi la classe des acétals et des cétals utilisés selon l'invention résultent de la condensation d'un aldéhyde ou d'une cètone avec un monoalcool ou un polyalcool, par exemple un glycol. Ils répondent à la formule générale suivante :

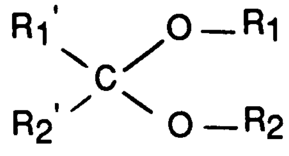

dans laquelle $R_1$' et $R_2$' sont constitués par un atome d'hydrogène ou un radical hydrocarbyl comprenant de 1 à 30 atomes de carbone, et $R_1$ et $R_2$ sont des radicaux hydrocarbyl comprenant de 1 à 30 atomes de carbone. Les deux radicaux $R_1$' et $R_2$' et les deux radicaux $R_1$ et $R_2$ peuvent être identiques ou différents. Ils peuvent également faire partie d'un cycle. On peut citer à titre d'exemples le diéthoxyméthane, le diisopropoxyméthane, le diéthoxy-1,1-éthane, le diisobutoxy-1,1-éthane, le diméthoxy-1,1-décane, le nonyl-2-dioxolane-1,3, le diméthoxy-2,2-propane, le dibutoxy-2,2-propane, le dioctoxy-2,2-propane, le diméthoxy-2,2-octane, le diméthoxy-1,1-cyclohexane, le di-(éthyl-2-hexyloxy)-2,2-propane.

Les composés de l'aluminium utilisés dans l'invention sont représentés par la formule générale $AlR''_nX_{3-n}$ dans laquelle R" est un radical hydrocarbyl, de préférence alkyl, comprenant de 1 à 6 atomes de carbone, X est un atome de chlore ou de brome, de préférence un atome de chlore, et n est un nombre compris entre 1 et 2, pouvant être égal notamment à 1, à 1,5 ou à 2.

On peut citer à titre d'exemples le chlorodiéthylaluminium, le dichloroéthylaluminium, le sesquichlorure d'éthylaluminium, ou leurs mélanges.

Les composants du catalyseur peuvent être mis en contact dans un ordre quelconque au sein d'un hydrocarbure, par exemple un hydrocarbure saturé comme l'hexane ou l'heptane et/ou un hydrocarbure aromatique comme le toluène, et/ou d'un ou des sous-produits d'oligomérisation tels que les oligomères supérieurs. De préférence, le composé de zirconium est d'abord mélangé avec l'acétal ou le cétal, puis le composé d'aluminium est ajouté à l'ensemble.

Le rapport molaire entre l'acétal ou le cétal et le composé de zirconium est d'environ 0,1:1 à 5:1 et de préférence d'environ 0,5:1 à 2:1. Le rapport molaire entre le composé d'aluminium et le composé de zirconium est d'environ 1:1 à 100:1, de préférence d'environ 5:1 à 50:1. La concentration de zirconium dans la solution catalytique ainsi préparée est avantageusement comprise entre $10^{-4}$ et 0,5 mole par litre, et de préférence entre $2.10^{-3}$ et 0,1 mole par litre. La température à laquelle se fait le mélange des trois composants est habituellement comprise entre -10 et +150 °C, de préférence entre 0 et +80 °C, et par exemple égale à la température ambiante (15 à 30 °C). Ce mélange peut être effectué sous une atmosphère de gaz inerte ou d'éthylène.

La solution catalytique ainsi obtenue peut être utilisée telle quelle ou elle peut être diluée par addition des produits de la réaction.

Dans un mode particulier de mise en oeuvre de la réaction catalytique d'oligomérisation en discontinu, on introduit un volume choisi de la solution catalytique, préparée comme décrit ci-dessus, dans un réacteur muni des habituels systèmes d'agitation et de refroidissement, puis on pressurise par de l'éthylène à une pression généralement comprise entre 0,5 et 15 MPa, de préférence entre 1 et 10 MPa ; on maintient la température en général entre 20 et 180 °C, de préférence entre 40 et 150 °C. On alimente le réacteur d'oligomérisation par de l'éthylène à pression constante jusqu'à ce que le volume total de liquide produit représente entre 2 et 50 fois le volume de la solution catalytique primitivement introduit. On détruit alors le catalyseur, par exemple par addition d'eau, et on soutire et sépare les produits de la réaction et le solvant éventuel.

En cas d'opération continue, la mise en oeuvre est de préférence la suivante : la solution catalytique est injectée en même temps que l'éthylène dans un réacteur agité par les moyens mécaniques classiques ou par une recirculation extérieure. On peut aussi injecter séparément les composants du catalyseur dans le milieu réactionnel, par exemple le produit d'interaction du composé de zirconium avec l'acétal (ou le cétal) d'une part, et l'halogènure d'hydrocarbyl-aluminium d'autre part. La température est maintenue entre 20 et 180 °C, de préférence entre 40 et 150 °C, et la pression est ajustée généralement entre 0,5 et 15 MPa. Par une vanne de détente, qui maintient la pression constante, s'écoule une partie du mélange réactionnel, à un débit massique égal au débit massique des fluides introduits. Le fluide ainsi détendu est envoyé dans un système de colonnes à distiller qui permet de séparer les oligomères de l'éthylène d'une part, éthylène qui peut être renvoyé au réacteur, puis les oligomères entre eux d'autre part. Les produits lourds contenant le catalyseur peuvent être incinérés.

Les exemples suivants illustrent l'invention sans toutefois en limiter la portée.

## EXEMPLE 1

Dans un autoclave en acier inoxydable d'un volume utile de 250 ml, muni d'une double enveloppe permettant de réguler la température par circulation d'eau, on introduit dans l'ordre, sous atmosphère d'argon et à la température ambiante : 0,2 x $10^{-3}$ mole du complexe $[(C_7H_{15}COO)_3Zr]_2O$, où $C_7H_{15}COO$ est un reste éthyl-2 hexanoate, 50 ml d'heptane, puis, au moyen d'une seringue hypodermique, 20,8 mg de diméthoxy-2,2-propane (soit 0,2 x $10^{-3}$ mole). Après quelques minutes, on introduit 2,4 x $10^{-3}$ mole de chlorodiéthylaluminium en solution dans 10 ml d'heptane.

La température est alors portée à 75 °C tout en introduisant dans l'autoclave de l'éthylène de manière à maintenir une pression constante de 6 MPa.

Après 2 heures de réaction, l'introduction d'éthylène est arrêtée et le catalyseur est détruit par injection sous pression de 2 ml d'eau. On a consommé au total 71 g d'éthylène.

La composition des produits est donnée dans le tableau 1. On recueille en outre 0,2 % en poids de polymère solide par rapport à l'éthylène consommé.

## EXEMPLE 2 (Comparatif)

Dans le même appareillage que celui qui a été utilisé dans l'exemple 1 et dans les mêmes conditions, à ceci près que le diméthoxy-2,2-propane a été omis, on a consommé au total 96,5 g d'éthylène au bout de 15 minutes de réaction. La composition des produits obtenus, donnée dans le tableau 1, montre l'effet bénéfique de la présence du cétal dans

le catalyseur sur la sélectivité en oléfines alpha légères. On a recueilli d'autre part 8,4 % de polymère solide par rapport à l'éthylène consommé, c'est-à-dire beaucoup plus que dans l'exemple 1.

**EXEMPLE 3**

Dans un ballon en verre de 100 ml placé sous atmosphère inerte, on transfère à l'abri de l'humidité 2 x $10^{-3}$ mole de tétrachlorure de zirconium sublimé, puis on injecte au moyen d'une seringue hypodermique 45 ml de toluène séché et désaéré. La suspension blanche est agitée à température ambiante au moyen d'un barreau magnétique, et on ajoute dans le ballon 2 x $10^{-3}$ mole de diméthoxy-1,1-décane en solution dans 5 ml de toluène. En quelques minutes, le chlorure de zirconium se dissout et la coloration de la solution homogène ainsi obtenue évolue du jaune clair, à l'orange puis au rouge foncé, indiquant la formation d'un complexe.

Dans le même autoclave que celui décrit dans l'exemple 1, on introduit dans l'ordre, sous atmosphère d'argon, et à la température ambiante, 5 ml de la solution de complexe préparée ci-dessus, soit 0,2 x $10^{-3}$ mole de zirconium, 50 ml d'heptane, puis 1,2 x $10^{-3}$ mole de sesquichlorure d'éthylaluminium $Al_2Et_3Cl_3$ en solution dans 10 ml d'heptane.

La température est alors portée à 95 °C tout en introduisant dans l'autoclave de l'éthylène de manière à maintenir une pression constante de 6 MPa.

Après 2 heures de réaction, l'introduction d'éthylène est arrêtée et le catalyseur est détruit par injection sous pression de 2 ml d'eau. On a consommé au total 51 g d'éthylène.

La composition des produits est donnée dans le tableau 1. On recueille seulement des traces de polymère solide, en quantité trop faible pour être mesurées avec précision.

**EXEMPLE 4**

Dans les mêmes appareillages et avec le même mode opératoire que décrits dans l'exemple 3, à ceci près qu'on a remplacé le diméthoxy-1,1-décane par le diisopropoxyméthane dans les mêmes proportions, la réaction d'oligomérisation à consommé 98,3 g d'éthylène en une heure.

La composition des produits est donnée dans le tableau 1. On n'a recueilli que des traces de polymère solide.

**EXEMPLE 5**

Dans les mêmes appareillages et avec le même mode opératoire que décrits dans l'exemple 3, à ceci près qu'on a remplacé le diméthoxy-1,1-décane par le diméthoxy-2,2-propane dans les mêmes proportions, la réaction d'oligomérisation à consommé 77,3 g d'éthylène en une heure.

La composition des produits est donnée dans le tableau 1. On n'a recueilli que des traces de polymère solide.

**EXEMPLE 6**

Dans les mêmes appareillages et avec le même mode opératoire que décrits dans l'exemple 3, à ceci près qu'on a remplacé le diméthoxy-1,1-décane par le dioctoxy-2,2-propane dans les mêmes proportions, la réaction d'oligomérisation a consommé 88,5 g d'éthylène en une heure.

La composition des produits est donnée dans le tableau 1. On n'a recueilli que des traces de polymère solide.

**EXEMPLE 7**

Dans les mêmes appareillages et avec le même mode opératoire que décrits dans l'exemple 3, à ceci près qu'on a remplacé le diméthoxy-1,1-décane par une quantité molaire double de diméthoxy-2,2-octane (c'est-à-dire 0,4 x $10^{-3}$ mole de cétal pour 0,2 x $10^{-3}$ mole de zirconium mis en oeuvre), la réaction d'oligomérisation à consommé 39,3 g d'éthylène en 3 heures.

La composition des produits est donnée dans le tableau 1. On n'a recueilli que des traces de polymère solide.

**EXEMPLE 8**

Dans les mêmes appareillages et avec le même mode opératoire que décrits dans l'exemple 3, à ceci près qu'on a remplacé le diméthoxy-1,1-décane par le dibutoxy-2,2-propane dans les mêmes proportions, et que le température de la réaction d'oligomérisation a été fixée à 65 °C au lieu de 95 °C, cette réaction a consommé 44 g d'éthylène en 2 heures.

La composition des produits est donnée dans le tableau 1. On n'a recueilli que des traces de polymère solide.

**EXEMPLE 9**

Dans les mêmes appareillages et avec le même mode opératoire que décrits dans l'exemple 3, à ceci près qu'on a remplacé le diméthoxy-2,2-décane par le dioctoxy-2,2-propane dans les mêmes proportions et qu'on a introduit 2,4 x $10^{-3}$ mole de chlorodiéthyl-aluminium au lieu de 1,2 x $10^{-3}$ mole de sesquichlorure d'éthylaluminium, la réaction d'oligomérisation a consommé 83,4 g d'éthylène en 30 minutes.

La composition des produits est donnée dans le tableau 1. On a recueilli en outre 0,47 % en poids de polymère solide par rapport à l'éthylène consommé.

**EXEMPLE 10**

Dans les mêmes appareillages et avec le même mode opératoire que décrits dans l'exemple 9, à ceci près qu'on a mis en oeuvre une quantité double de dioctoxy-2,2-propane (c'est-à-dire 0,4 x $10^{-3}$ mole de cétal pour 0,2 x $10^{-3}$ mole de zirconium), la réaction d'oligomérisation a consommé 58,9 g d'éthylène en 2 heures.

La composition des produits est donnée dans le tableau 1. On a recueilli en outre 1,35 % en poids de polymère solide par rapport à l'éthylène consommé.

**EXEMPLE 11**

Dans les mêmes appareillage et avec le même mode opératoire que décrits dans l'exemple 3, à ceci près qu'on a remplacé le diméthoxy-2,2-décane par le diisopropoxyméthane dans les même proportions, qu'on a introduit 2,4 x $10^{-3}$ mole de chlorodiéthylaluminium au lieu de 1,2 x $10^{-3}$ mole de sesquichlorure d'éthyl-aluminium, et que la réaction d'oligomérisation a été effectuée à 70 °C au lieu de 95 °C, cette réaction a consommé 69 g d'éthylène en une heure.

La composition des produits est donnée dans le tableau 1. On a recueilli en outre 0,86 % en poids de polymère solide par rapport à l'éthylène consommé.

**EXEMPLE 12 (Comparatif)**

Dans les mêmes appareillages et avec le même mode opératoire que décrits dans l'exemple 3, à ceci près qu'on a omis d'introduire le diméthoxy-1,1-décane (le tétrachlorure de zirconium ayant donc été introduit dans l'autoclave à l'état de suspension), la réaction a consommé 32,8 g d'éthylène en 4 heures.

La composition des produits donnée dans le tableau 1 montre une mauvaise sélectivité pour les oléfines alpha légères. On recueille en outre une quantité importante de polymère, égale à 15 % en poids par rapport à l'éthylène consommé.

Cet exemple illustre la double amélioration apportée par l'introduction d'un acétal, concernant la sélectivité pour les oléfines alpha légères, et la réduction du taux relatif de polymère sous-produit.

## TABLEAU 1

| EXEMPLE | Répartition des produits obtenus (% poids *) | | | | | Teneur en oléfine alpha (% poids) | |
|---|---|---|---|---|---|---|---|
| | $C_4$ | $C_6$ | $C_8$ | $C_{10}$ | $C_{12}^+$ | dans $C_4$ | dans $C_6$ |
| 1 | 44,1 | 31,2 | 14,1 | 6,1 | 4,4 | >99 | 94,9 |
| 2 | 30,6 | 24,1 | 15,6 | 10,0 | 11,3 | 99,6 | 88,5 |
| 3 | 22,3 | 24,6 | 18,2 | 12,2 | 23,2 | 98,9 | 97,8 |
| 4 | 17,0 | 20,8 | 18,0 | 13,7 | 31,1 | 99,5 | 98,0 |
| 5 | 20,9 | 23,9 | 19,0 | 13,3 | 23,2 | 99,0 | 96,6 |
| 6 | 18,4 | 21,1 | 17,8 | 14,0 | 29,2 | 99,6 | 96 |
| 7 | 39,1 | 29,3 | 15,1 | 7,6 | 9,9 | 97,6 | 93,7 |
| 8 | 25,7 | 23,6 | 17,1 | 12,0 | 22,2 | 99,0 | 96,7 |
| 9 | 28,9 | 28,1 | 18,8 | 10,4 | 13,7 | 99,8 | 96,3 |
| 10 | 33,5 | 28,4 | 16,9 | 9,0 | 11,3 | 99,8 | 98,0 |
| 11 | 42,7 | 31,6 | 14,7 | 6,3 | 4,6 | 99,6 | 98,5 |
| 12 | 11,0 | 12,0 | 11,5 | 9,5 | 41,0 | >99 | 98,0 |

* le complément à 100 correspond au polymère formé.

### Revendications

1. Procédé de conversion de l'éthylène en oléfines alpha-légères dans lequel l'éthylène est mis au contact d'un catalyseur caractérisé en ce que ledit catalyseur est obtenu par mélange :

- d'un composé de zirconium de formule $ZrX_xY_yO_z$ dans laquelle X est un atome de chlore ou de brome, Y est un radical choisi dans le groupe formé par les alkoxy RO-, les amido $R_2N$-, les carboxylates RCOO-, où R est un radical hydrocarbyl comprenant de 1 à 30 atomes de carbone, x et y peuvent prendre les valeurs entières de 0 à 4 et z est égal à 0 ou 0,5, la somme x + y + 2z étant égale à 4,

- avec un composé organique de formule

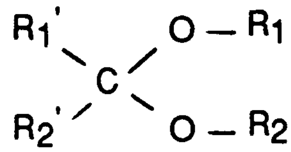

dans laquelle $R'_1$ et $R'_2$ sont constitués par un atome d'hydrogène ou un radical hydrocarbyl comprenant de 1 à 30 atomes de carbone, $R_1$ et $R_2$ sont des radicaux hydrocarbyl comprenant de 1 à 30 atomes de carbone,

- et avec un composé d'aluminium de formule $AlR''_n X_{3-n}$ dans laquelle R" est un radical hydrocarbyl comprenant de 1 à 6 atomes de carbone, X est un atome de chlore ou de brome, et n est un nombre compris entre 1 et 2.

**2.** Procédé selon la revendication 1, caractérisé en ce que le composé de zirconium et le composé organique sont mélangés, le produit obtenu étant ensuite mélangé avec le composé d'aluminium.

**3.** Procédé selon l'une des revendications 1 ou 2, caractérisé en ce que le composé organique est choisi dans le groupe formé par le diéthoxyméthane, le diisopropoxyméthane, le diethoxy-1,1 éthane, le diisobutoxy-1,1 éthane, le diméthoxy-1,1 décane, le nonyl-2 dioxolane-1,3, le diméthoxy-2,2 propane, le dibutoxy-2,2 propane, le dioctoxy-2,2 propane, le diméthoxy-2,2 octane, le diméthoxy-1,1 cyclohexane, le di-(éthyl-2-hexyloxy)-2,2-propane.

**4.** Procédé selon l'une des revendications 1 à 3, caractérisé en ce que le composé de zirconium est choisi dans le groupe formé par le tétrabromure de zirconium, le tétrapropylate de zirconium, le tétrabutylate de zirconium, le tétra-éthyl-2 hexanoate de zirconium et l'oxo-hexaéthyl-2 hexanoate de dizirconium.

**5.** Procédé selon l'une des revendications 1 à 3, caractérisé en ce que le composé de zirconium est le tétrachlorure de zirconium.

**6.** Procédé selon l'une des revendications 1 à 5, caractérisé en ce que le composé d'aluminium est choisi dans le groupe formé par le chlorodiéthylaluminium, le sesquichlorure d'éthylaluminium et leurs mélanges.

**7.** Procédé selon l'une des revendications 1 à 6, caractérisé en ce que le rapport molaire entre le composé organique et le composé de zirconium est compris entre 0,1:1 et 5:1.

**8.** Procédé selon l'une des revendications 1 à 7, caractérisé en ce que le rapport molaire entre le composé d'aluminium et le composé de zirconium est compris entre 1:1 et 100:1.

**9.** Procédé selon l'une des revendications 1 à 8 caractérisé en ce que le mélange du composé de zirconium avec le composé organique et avec le composé d'aluminium s'effectue à une température comprise entre 0 et 80 °C et sous une atmosphère de gaz inerte ou d'éthylène.

**10.** Procédé selon l'une des revendications 1 à 9 caractérisé en ce que la conversion de l'éthylène en oléfines alpha légères s'effectue à une température comprise entre 20 et 180 °C et sous une pression de 0,5 à 15 MPa.

**11.** Catalyseur obtenu par mélange :

- d'un composé de zirconium de formule $ZrX_xY_yO_z$ dans laquelle X est un atome de chlore ou de brome, Y est un radical choisi dans le groupe formé par les alkoxy RO-, les amido $R_2N$-, les carboxylates RCOO-, où R est un radical hydrocarbyl comprenant de 1 à 30 atomes de carbone, x et y peuvent prendre les valeurs entières de 0 à 4 et z est égal à 0 ou 0,5, la somme $x + y + 2z$ étant égale à 4,

- avec un composé organique de formule

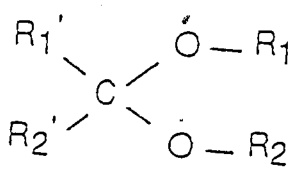

dans laquelle $R'_1$ et $R'_2$ sont constitues par un atome d'hydrogène ou un radical hydrocarbyl comprenant de 1 à 30 atomes de carbone, $R_1$ et $R_2$ sont des radicaux hydrocarbyl comprenant de 1 à 30 atomes de carbone,

- et avec un composé d'aluminium de formule $AlR''_n X_{3-n}$ dans laquelle R" est un radical hydrocarbyl comprenant de 1 à 6 atomes de carbone, X est un atome de chlore ou de brome, et n est un nombre compris entre 1 et 2.

**12.** Catalyseur selon la revendication 11, caractérisé en ce que le composé de zirconium et le composé organique sont mélangés, le produit obtenu étant ensuite mélangé avec le composé d'aluminium.

**13.** Catalyseur selon l'une des revendications 11 ou 12, caractérisé en ce que le composé organique est choisi dans le groupe formé par le diéthoxyméthane, le diisopropoxyméthane, le diethoxy-1,1éthane, le diisobutoxy-1,1éthane, le diméthoxy-1,1décane, le nonyl-2 dioxolane-1,3, le diméthoxy-2,2 propane, le dibutoxy-2,2 propane, le dioctoxy-2,2 propane, le diméthoxy-2,2 octane, le diméthoxy-1,1 cyclohexane, le di-(éthyl-2-hexyloxy)-2,2-propane.

**14.** Catalyseur selon l'une des revendications 11 à 13, caractérisé en ce que le composé de zirconium est choisi dans le groupe formé par le tétrabromure de zirconium, le tétrapropylate de zirconium, le tétrabutylate de zirconium, le tétra-éthyl-2 hexanoate de zirconium et l'oxo-hexaéthyl-2 hexanoate de dizirconium.

**15.** Catalyseur selon l'une des revendications 11 à 13, caractérisé en ce que le composé de zirconium est le tétrachlorure de zirconium.

**16.** Catalyseur selon l'une des revendications 11 à 15, caractérisé en ce que le composé d'aluminium est choisi dans le groupe formé par le chlorodiéthylaluminium, le sesquichlorure d'éthylaluminium et leurs mélanges.

**17.** Catalyseur selon l'une des revendications 11 à 16, caractérisé en ce que le rapport molaire entre le composé organique et le composé de zirconium est compris entre 0,1:1 et 5:1.

**18.** Catalyseur selon l'une des revendications 11 à 17, caractérisé en ce que le rapport molaire entre le composé d'aluminium et le composé de zirconium est compris entre 1:1 et 100:1.

**19.** Catalyseur selon l'une des revendications 11 à 18 caractérisé en ce que le mélange du composé de zirconium avec le composé organique et avec le composé d'aluminium s'effectue à une température comprise entre 0 et 80 °C et sous une atmosphère de gaz inerte ou d'éthylène.

## Claims

**1.** Process for the conversion of ethylene into light alpha olefins, in which ethylene is contacted with a catalyst, characterized in that said catalyst is obtained by mixing a zirconium compound of formula $ZrX_xY_yO_z$, in which X is a chlorine or bromine atom, Y is a radical chosen from within the group formed by RO- alkoxy, $R_2N$- amido and RCOO-carboxylate groups, in which R is a hydrocarby radical having 1 to 30 carbon atoms, in which x and y can assume integral values of 0 to 4 and z is equal to 0 or 0.5, the sum $x + y + 2z$ being equal to 4, with an organic compound of formula

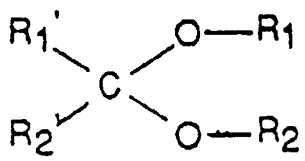

in which $R'_1$ and $R'_2$ are constituted by a hydrogen atom or a hydrocarbyl radical having 1 to 30 carbon atoms and $R_1$ and $R_2$ are hydrocarbyl radicals having 1 to 30 carbon atoms and with an aluminum compound of formula $AlR''_nX_{3-n}$ in which R" is a hydrocarbyl radical having 1 to 6 carbon atoms, X is a chlorine or bromine atom and n is a number between 1 and 2.

2. Process according to claim 1, characterized in that the zirconium compound and the organic compound are mixed, the product obtained then being mixed with the aluminum compound.

3. Process according to one of the claims 1 or 2, characterized in that the organic compound is chosen from within the group formed by diethoxy methane, diisopropoxy methane, 1,1-diethoxy ethane, 1,1-diisobutoxy ethane, 1,1-dimethoxy decane, 2-nonyl-1,3-dioxolan, 2,2-dimethoxy propane, 2,2-dibutoxy propane, 2,2-dioctoxy propane, 2,2-dimethoxy octane 1,1-dimethoxy cyclohexane, and di-(ethyl-2-hexyloxy)-2,2-propane.

4. Process according to any one of the claims 1 to 3, characterized in that the zirconium compound is chosen from within the group formed by zirconium tetrabromide, zirconium tetrapropylate, zirconium tetrabutylate, zirconium-2-tetraethyl hexanoate and dizirconium-2-oxohexaethyl hexanoate.

5. Process according to any one of the claims 1 to 3, characterized in that the zirconium compound is zirconium tetrachloride.

6. Process according to any one of the claims 1 to 5, characterized in that the aluminium compound is chosen from within the group formed by chlorodiethyl aluminum, ethyl aluminum sesquichloride and mixtures thereof.

7. Process according to any one of the claims 1 to 6, characterized in that the molar ratio between the organic compound and the zirconium compound is between 0.1:1 and 5:1.

8. Process according to any one of the claims 1 to 7, characterized in that the molar ratio between the aluminium compound and the zirconium compound is between 1:1 and 100:1.

9. Process according to any one of the claims 1 to 8, characterized in that the mixing of the zirconium compound with the organic compound and with the aluminium compound takes place at a temperature between 0 and 80°C and under an ethylene or inert gas atmosphere.

10. Process according to any one of the claims 1 to 9, characterized in that the conversion of ethylene into light alpha olefins takes place at a temperature between 20 and 180°C and under a pressure of 0.5 to 15 MPa.

11. Catalyst obtained by mixing a zirconium compound of formula $ZrX_xY_yO_z$, in which X is a chlorine or bromine atom, Y is a radical chosen from within the group formed by RO- alkoxy, $R_2N$- amido and RCOO-carboxylate groups, in which R is a hydrocarbyl radical having 1 to 30 carbon atoms, in which x and y can assume integral values of 0 to 4, and z is equal to 0 or 0.5, the sum $x + y + 2z$ being equal to 4, with an organic compound of formula

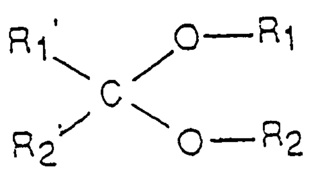

in which $R'_1$ and $R'_2$ are constituted by a hydrogen atom or a hydrocarbyl radical having 1 to 30 carbon atoms and $R_1$ and $R_2$ are hydrocarbyl radicals having 1 to 30 carbon atoms and with an aluminium compound of formula $AlR''_nX_{3-n}$ in which R'' is a hydrocarbyl radical having 1 to 6 carbon atoms, X is a chlorine or bromine atom and n is a number between 1 and 2.

**12.** Catalyst according to claim 11, characterized in that the zirconium compound and the organic compound are mixed, the product obtained then being mixed with the aluminium compound.

**13.** Catalyst according to one of the claims 11 or 12, characterized in that the organic compound is chosen from within the group formed by diethoxy methane, diisopropoxy methane, 1,1-diethoxy ethane, 1,1-diisobutoxy ethane, 1,1-dimethoxy decane, 2-nonyl-1,3-dioxolan, 2,2-dimethoxy propane, 2,2-dibutoxy propane, 2,2-dioctoxy propane, 2,2-dimethoxy octane, 1,1-dimethoxy cyclohexane, and di-(ethyl-2-hexyloxy)-2,2-propane.

**14.** Catalyst according to any one of the claims 11 to 13, characterized in that the zirconium compound is chosen from within the group formed by zirconium tetrabromide, zirconium tetrapropylate, zirconium tetrabutylate, zirconium-2-tetraethyl hexanoate and dizirconium-2-oxohexaethyl hexanoate.

**15.** Catalyst according to any one of the claims 11 to 13, characterized in that the zirconium compound is zirconium tetrachloride.

**16.** Catalyst according to any one of the claims 11 to 15, characterized in that the aluminum compound is chosen from within the group formed by chlorodiethyl aluminum, ethyl aluminium sesquichlorde and mixtures thereof.

**17.** Catalyst according to any one of the claims 11 to 16, characterized in that the molar ratio between the organic compound and the zirconium compound is between 0.1:1 and 5:1.

**18.** Catalyst according to any one of the claims 11 to 17, characterized in that the molar ratio between the aluminium compound and the zirconium compound is between 1:1 and 100:1.

**19.** Catalyst according to any one of the claims 11 to 18, characterized in that the mixing of the zirconium compound with the organic compound and with the aluminium compound takes place at a temperature between 0 and 80°C and under an ethylene or inert gas atmosphere.

**Patentansprüche**

**1.** Verfahren zum Umsetzen von Ethylen in leichte α-Olefine, bei dem das Ethylen in Kontakt mit einem Katalysator gebracht wird, dadurch gekennzeichnet, daß der Katalysator erhalten wird durch Mischen:

- einer Zirkoniumverbindung der Formel $ZrX_xY_yO_z$, bei der X ein Chlor- oder Bromatom, Y ein Radikal ausgewählt aus der durch Alkoxy RO-, Amido $R_2N$-, Carboxylate RCOO- gebildeten Gruppe ist, wo R ein Kohlenwasserstoffradikal mit 1 bis 30 Kohlenstoffatomen ist, x und y ganzzahlige Werte von 0 bis 4 annehmen können und z gleich 0 oder 0,5 ist, wobei die Summe x + y + 2z gleich 4 ist,

- mit einer organischen Verbindung der Formel

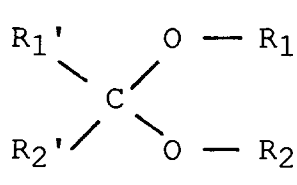

wobei $R'_1$ und $R'_2$ aus einem Wasserstoffatom oder einem Kohlenwasserstoffradikal mit 1 bis 30 Kohlenstoffatomen gebildet sind und $R_1$ und $R_2$ Kohlenwasserstoffradikale mit 1 bis 30 Kohlenstoffatomen sind,

- und mit einer Aluminiumverbindung der Formel $AlR''_nX_{3-n}$, bei der R'' ein Kohlenwasserstoffradikal mit 1 bis 6 Kohlenstoffatomen ist, X ein Chlor- oder Bromatom ist und n eine Zahl zwischen 1 und 2 ist.

2. Verfahren nach Anspruch 1, dadurch gekennzeichnet, daß die Zirkoniumverbindung und die organische Verbindung gemischt werden und das erhaltene Produkt anschließend mit der Aluminiumverbindung gemischt wird.

3. Verfahren nach einem der Ansprüche 1 oder 2, dadurch gekennzeichnet, daß die organische Verbindung ausgewählt wird aus der Gruppe, die gebildet ist durch Diethoxymethan, Diisopropoxymethan, Diethoxy-1,1-ethan, Diisobutoxy-1,1-ethan, Dimethoxy-1,1-decan, Nonyl-2-dioxolan-(1,3), Dimethoxy-2,2-propan, Dibutoxy-2,2-propan, Dioctoxy-2,2-propan, Dimethoxy-2,2-octan, Dimethoxy-1,1-cyclohexan, Di-(ethyl-2-hexyloxy)-2,2-propan.

4. Verfahren nach einem der Ansprüche 1 bis 3, dadurch gekennzeichnet, daß die Zirkoniumverbindung ausgewählt wird aus der Gruppe gebildet durch Zirkoniumtetrabromid, Zirkoniumtetrapropylat, Zirkoniumtetrabutylat, Zirkoniumtetraethyl-2-hexanoat und Dizirkoniumoxo-hexaethyl-2-hexanoat.

5. Verfahren nach einem der Ansprüche 1 bis 3, dadurch gekennzeichnet, daß die Zirkoniumverbindung Zirkoniumtetrachlorid ist.

6. Verfahren nach einem der Ansprüche 1 bis 5, dadurch gekennzeichnet, daß die Aluminiumverbindung ausgewählt ist aus der Gruppe, die gebildet ist durch Aluminiumdiethylchlorid, Aluminiumethylsesquichlorid und deren Gemische.

7. Verfahren nach einem der Ansprüche 1 bis 6, dadurch gekennzeichnet, daß das molare Verhältnis zwischen der organischen Verbindung und der Zirkoniumverbindung zwischen 0,1:1 und 5:1 liegt.

8. Verfahren nach einem der Ansprüche 1 bis 7, dadurch gekennzeichnet, daß das molare Verhältnis zwischen der Aluminiumverbindung und der Zirkoniumverbindung zwischen 1:1 und 100:1 liegt.

9. Verfahren nach einem der Ansprüche 1 bis 8, dadurch gekennzeichnet, daß das Mischen der Zirkoniumverbindung mit der organischen Verbindung und mit der Aluminiumverbindung bei einer Temperatur zwischen 0 und 80°C und unter einer Inertgas- oder Ethylenatmosphäre durchgeführt wird.

10. Verfahren nach einem der Ansprüche 1 bis 9, dadurch gekennzeichnet, daß die Umsetzung des Ethylens in leichte $\alpha$-Olefine bei einer Temperatur zwischen 20 und 180°C und unter einem Druck von 0,5 bis 15 MPa durchgeführt wird.

11. Katalysator, erhalten durch Mischen:

- einer Zirkoniumverbindung der Formel $ZrX_xY_yO_z$, bei der X ein Chlor- oder Bromatom, Y ein Radikal ausgewählt aus der durch Alkoxy RO-, Amido $R_2N$-, Carboxylate RCOO- gebildeten Gruppe ist, wo R ein Kohlenwasserstoffradikal mit 1 bis 30 Kohlenstoffatomen ist, x und y ganzzahlige Werte von 0 bis 4 annehmen können und z gleich 0 oder 0,5 ist, wobei die Summe x + y + 2z gleich 4 ist,
- mit einer organischen Verbindung der Formel

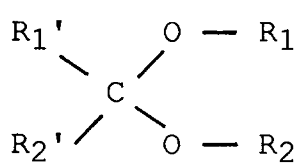

wobei $R'_1$ und $R'_2$ aus einem Wasserstoffatom oder einem Kohlenwasserstoffradikal mit 1 bis 30 Kohlenstoffatomen gebildet sind, $R_1$ und $R_2$ Kohlenwasserstoffradikale mit 1 bis 30 Kohlenstoffatomen sind,

- und mit einer Aluminiumverbindung der Formel $AlR''_nX_{3-n}$, bei der R'' ein Kohlenwasserstoffradikal mit 1 bis 6 Kohlenstoffatomen ist, X ein Chlor- oder Bromatom ist und n eine Zahl zwischen 1 und 2 ist.

12. Katalysator nach Anspruch 11, dadurch gekennzeichnet, daß die Zirkoniumverbindung und die organische Verbindung gemischt werden und das erhaltene Produkt anschließend mit der Aluminiumverbindung gemischt wird.

13. Katalysator nach einem der Ansprüche 11 oder 12, dadurch gekennzeichnet, daß die organische Verbindung ausgewählt ist aus der Gruppe, die gebildet ist durch Diethoxymethan, Diisopropoxymethan, Diethoxy-1,1-ethan, Diisobutoxy-1,1-ethan, Dimethoxy-1,1-decan, Nonyl-2-dioxolan-(1,3), Dimethoxy-2,2-propan, Dibutoxy-2,2-propan, Dioctoxy-2,2-propan, Dimethoxy-2,2-octan, Dimethoxy-1,1-cyclohexan, Di-(ethyl-2-hexyloxy)-2,2-propan.

14. Katalysator nach einem der Ansprüche 11 bis 13, dadurch gekennzeichnet, daß die Zirkoniumverbindung ausgewählt ist aus der Gruppe gebildet durch Zirkoniumtetrabromid, Zirkoniumtetrapropylat, Zirkoniumtetrabutylat, Zirkoniumtetraethyl-2-hexanoat und Dizirkoniumoxo-hexaethyl-2-hexanoat.

15. Katalysator nach einem der Ansprüche 11 bis 13, dadurch gekennzeichnet, daß die Zirkoniumverbindung Zirkoniumtetrachlorid ist.

16. Katalysator nach einem der Ansprüche 11 bis 15, dadurch gekennzeichnet, daß die Aluminiumverbindung ausgewählt ist aus der Gruppe, die gebildet ist durch Aluminiumdiethylchlorid, Aluminiumethylsesquichlorid und deren Gemische.

17. Katalysator nach einem der Ansprüche 11 bis 16, dadurch gekennzeichnet, daß das molare Verhältnis zwischen der organischen Verbindung und der Zirkoniumverbindung zwischen 0,1:1 und 5:1 liegt.

18. Katalysator nach einem der Ansprüche 11 bis 17, dadurch gekennzeichnet, daß das molare Verhältnis zwischen der Aluminiumverbindung und der Zirkoniumverbindung zwischen 1:1 und 100:1 liegt.

19. Katalysator nach einem der Ansprüche 11 bis 18, dadurch gekennzeichnet, daß das Mischen der Zirkoniumverbindung mit der organischen Verbindung und mit der Aluminiumverbindung bei einer Temperatur zwischen 0 und 80°C und unter einer Inertgas- oder Ethylenatmosphäre durchgeführt wird.